(19) Europäisches Patentamt
European Patent Office
Office européen des brevets

(11) Numéro de publication: 0 115 227
B1

(12) **FASCICULE DE BREVET EUROPÉEN**

(45) Date de publication du fascicule du brevet: **18.03.87**

(51) Int. Cl.⁴: **C 07 K 7/06**, A 61 K 37/02

(21) Numéro de dépôt: **83402404.4**

(22) Date de dépôt: **13.12.83**

(54) **Nouveaux dérivés peptidiques, leur procédé de fabrication et leur utilisation pharmaceutique.**

(30) Priorité: **16.12.82 FR 8221127**

(43) Date de publication de la demande: **08.08.84 Bulletin 84/32**

(45) Mention de la délivrance du brevet: **18.03.87 Bulletin 87/12**

(84) Etats contractants désignés: **BE CH DE GB IT LI**

(56) Documents cité:
**GB-A-1 063 728**

**CHEMICAL ABSTRACTS, vol. 93, no. 21, 1980, réf. no. 198289n, page 115, Columbus Ohio (US)**
**CHEMICAL ABSTRACTS, vol. 69, 1968, réf. no. 49624v, page 4632, Columbus Ohio (US)**

**Le dossier contient des informations techniques présentées postérieurement au dépôt de la demande et ne figurant pas dans le présent fascicule.**

(73) Titulaire: **COMMISSARIAT A L'ENERGIE ATOMIQUE Etablissement de Caractère Scientifique Technique et Industriel, 31/33, rue de la Fédération, F-75015 Paris (FR)**

(72) Inventeur: **Charon, Marie- Hélène, 18, rue du Printemps, F-75017 Paris (FR)**
Inventeur: **Fromageot, Pierre, 6, Avenue de la Reine Amélie, F-78150 Le Chesnay (FR)**
Inventeur: **Pham Van Chuong, Paul, 65, rue des Marguerites, F-92160 Antony (FR)**

(74) Mandataire: **Mongrédien, André, c/o BREVATOME 25, rue de Ponthieu, F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 115 227 B1

LIBER, STOCKHOLM 1987

**Description**

La présente invention a pour objet de nouveaux derives peptidiques, leur procédé de préparation et leur utilisation pharmaceutique.

De façon plus précise, elle concerne des dérivés peptidiques de la pentagastrine utilisables notamment comme antagonistes vis-à-vis de la gastrine et des "polypeptides apparentés".

On rappelle que la gastrine est une hormone naturelle connue en particulier pour être responsable de la sécrétion du suc gastrique. Son mode d'action sur les organes de l'appareil digestif est lié, comme celui de la plupart des hormones, à la présence dans ces organes de cellules-cibles dont les membranes plasmiques comportent des récepteurs biologiques spécifiques, susceptibles de fixer les molécules d'hormones et d'engendrer sous l'action de ces dernières une cascade de réactions biochimiques aboutissant à la production d'un produit particulier tel que l'acide chlorhydrique.

On définit, par ailleurs, sous le terme de "polypeptides apparentés", une famille de polypeptides présentant à leur extrémité C-terminale la séquence des quatre acides aminés: L-tryptophanyl-L-méthionyl-L-aspartyl-L-phénylalanine amide, caractéristique de l'activité biologique de la gastrine; ces polypeptides sont de ce fait susceptibles également d'engendrer des réactions biochimiques en se fixant sur des récepteurs spécifiques.

On sait qu'une hypersécrétion hormonale peut entraîner certains troubles pathologiques connus, par exemple pour la gastrine, au cours du syndrome de Zollinger-Ellison et suspectés dans de nombreuses autres affections.

Aussi, depuis quelques années, les recherches ont porté sur la préparation de composés capables d'inhiber la sécrétion gastrique. Dans ce but, on a proposé des polypeptides jouant le rôle d'antagonistes vis-à-vis de la pentagastrine, tels que les composés de formule A-W-X-Asp-Y-NHR dans laquelle A est l'hydrogène, un groupe alcanoyle ayant de 2 à 6 atomes de carbone, un groupe succinyle, t-butoxy-carbonyle, benzyloxycarbonyle, D-pyroglutamyle ou L-pyroglutamyle; W est le radical tryptophanyle ou D-tryptophanyle; X est le radical méthionyle, D-méthionyle, norleucyle ou D-norleucyle; Y est un radical de formule:

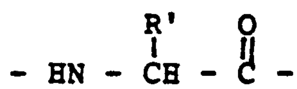

dans laquelle R' est un radical alkyle en $C_1$ à $C_6$ ou un radical alkyle cyclo-alkyle en $C_6$ à $C_7$, ou de formule:

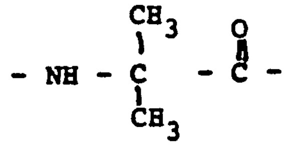

et R est l'hydrogène ou un radical alkyle en $C_1$ à $C_6$.

Dans ces polypeptides, par exemple le N-t-butoxycarbonyl-L-tryptophanyl-L-méthionyl-L-aspartyl-D-alanine amide, le remplacement du radical L-phénylalanyle de la séquence des quatre acides aminés de la gastrine par le radical:

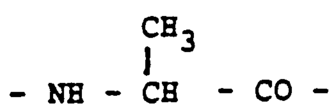

permet d'obtenir cet effet inhibiteur (voir brevet américain n° 4.012.367 de Robert H. Mazur).

On a également utilisé comme inhibiteur de la sécrétion gastrique, des polypeptides comportant la séquence des quatre acides aminés de la gastrine, mais dont le résidu tryptophanyle a été substitué par le radical orthonitrophénylthio, comme cela est décrit dans le brevet français N° 2.364.659 déposé le 21/9/76 au nom du Commissariat à l'Energie Atomique et de l'INSERM. Ainsi, jusqu'à présent, on a pu obtenir cet effet inhibiteur de la sécrétion gastrique, soit en modifiant la séquence caractéristique des quatre acides amines de la gastrine, soit en substituant le résidu tryptophanyle de cette séquence.

A la suite d'autres recherches, on a maintenant trouvé que l'on pouvait obtenir également un effet inhibiteur en modifiant le résidu aspartyle de la séquence des quatre acides aminés de la gastrine.

La présente invention a précisément pour objet de nouveaux dérivés peptidiques comportant sensiblement la séquence des quatre acides aminés de la gastrine et présentant un effet inhibiteur de la sécrétion gastriquer grâce à la modification du résidu aspartyle.

Le dérivé peptidique de l'invention répond à la formule suivante:

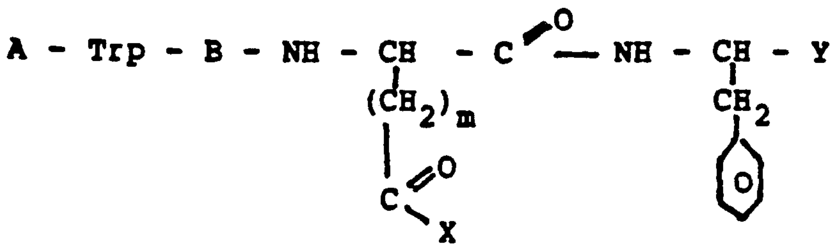

(I)

dans laquelle:

- A représente l'hydrogène; un radical dérivé d'un acide aminé choisi parmi les radicaux D ou L-pyroglutamyle, L ou D-alanyle, B-alanyle, glycyle, L ou D-prolyle, L ou D-valyle, L ou D-phénylalanyle, L ou D-homocystéyle, L ou D-aspartyle, L ou D-glutamyle, L ou D-histidyle, L ou D-méthionyle, L ou D-thréonyle, L ou D-séryle, L ou D-cystéyle, L ou D-leucyle, L ou D-isoleucyle, L ou D-arginyle, L ou D-tryptophanyle, L ou D-tyrosyle, L ou D-lysyle et L ou D-ornithyle, un groupement de formule DE où D représente le radical N-t-butyloxy-carbonyle (BOC), tert-amyloxycarbonyle (tAOC), N-benzyloxycarbonyle, N-benzoyle N-acétyle, N-pivaloyle, N-carbamoyle ou N-succinyle et E représente une simple liaison, un radical dérivé d'un acide amine choisi parmi les radicaux D ou L-pyroglutamyle, L ou D-alanyle, B-alanyle, glycyle, L ou D-prolyle, L ou D-valyle, L ou D-phénylalanyle, L ou D-homocystéyle, L ou D-aspartyle, L ou D-glutamyle, L ou D-histidyle, L ou D-méthionyle, L ou D-thréonyle, L ou D-séryle, L ou D-cystéyle, L ou D-leucyle, L ou D-isoleucyle, L ou D-arginyle, L ou D-tryptophanyle, L ou D-tyrosyle, L ou D-lysyle et L ou D-ornithyle, cet acide aminé étant non substitué ou substitué par $SO_3H$, ou un radical dérivé d'un peptide formé de 2 à 5 acides aminés choisis parmi (L ou D)-glutamyl-(L ou D)-glutamyl-(L ou D)-alanyl-(L ou D)-tyrosylglycyle, (L ou D)-alanyl-(L ou D)-phénylalanyl-(L ou D)-isoleucyl-glycyle, (L ou D)-alanyl-(L ou D)-tyrosylglycyle, (L ou D)-lysylglycyle, (L ou D)-tyrosyl-B-alanyle, (L ou D)-tyrosyl-(L ou D)-méthionylglycyle, (L ou D)-tyrosyl-(L ou D)-thréonylglycyle, (L ou D)-tyrosyl (O-sulfate)-(L ou D)-méthionylglycyle et (L ou D)-tyrosyl (O-sulfate)-(L ou D)-thréonylglycyle, ce peptide étant non substitué ou substitué par $SO_3H$;
- B représente le radical L-méthionyle, D-méthionyle, L-norleucyle, D-norleucyle, L-leucyle, D-leucyle, L-norvalyle ou D-norvalyle;
- Y représente H, $CH_2OH$, $COOR^1$ avec $R^1$ représentant l'hydrogène ou un radical alkyle en $C_1$ à $C_4$ ou $CONHR^2$ avec $R^2$ representant l'hydrogène, un radical alkyle en $C_1$ à $C_4$ ou $NH_2$;
- m est un nombre allant de 0 à 6; et
- X représente un radical de formule:

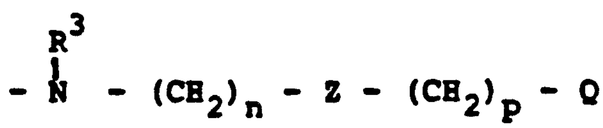

où n est un nombre allant de 1 à 8, p est un nombre allant de 0 à 3, $R^3$ représente H ou $CH_3$, Z représente une simple liaison ou S, et Q représente un radical choisi parmi les radicaux de formule:

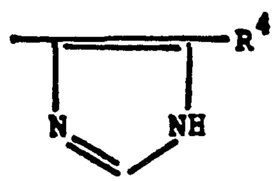

avec $R^4$ représentant H ou un radical alkyle en $C_1$ à $C_4$,

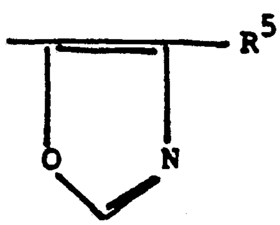 ; 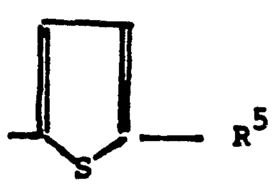 ; 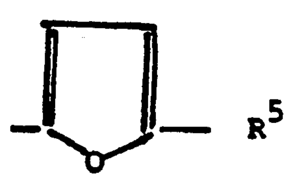

avec $R^5$ représentant H, un radical alkyle en $C_1$ à $C_4$ ou

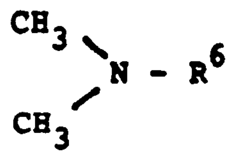

avec $R^6$ représentant un radical alkylène en $C_1$ à $C_3$; et

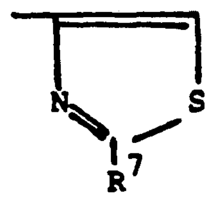

avec $R^7$ représentant H, un radical alkyle en $C_1$ à $C_4$,

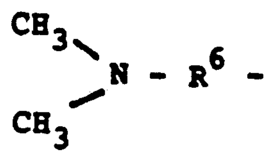

avec $R^6$ ayant la signification donnée ci-dessus, ou

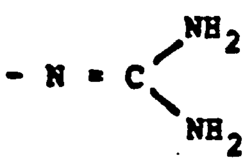

A titre d'exemple, A peut représenter les radicaux suivants: pyroglutamyle, N-acétyle, N-benzoyle, N-t-butyloxycarbonyle, glycyle, N-benzoylglycyle, N-benzyloxycarbonylglycyle, β-alanyle, N-acétyl-β-alanyle, N-t-butyloxycarbonyl-β-alanyle, N-benzoyl-β-alanyle, N-benzyloxycarbonyl-β-alanyle, N-pivaloyl-β-alanyle, N-t-butyloxycarbonyl-L ou D-alanyle, N-benzyloxycarbonyl-L ou D-prolyle, N-t-butyloxycarbonyl-L ou D-homocystéinyle, N-benzyl oxycarbonyl-L ou D-valyle, N-benzyloxycarbonyl-L ou D-phénylalanyle, N -acétyl-γ-aminobutyryle, N-α-benzyloxycarbonyl-L ou D-lysyle, N-ε-benzyloxycarbonyl-L ou D-lysyle, N-α-benzyloxycarbonyl-L ou D-lysylglycyle, N-t-butyloxycarbonyl-L ou D-alanyl-L ou D tyrosylglylcyle, N-benzyloxycarbonyl-L ou D-glutamyl-L ou D-glutamyl-L ou D-alanyl-L ou D-tyrosylglycyle et N-t-butyloxycarbonyl-L ou D-alanyl-L ou D-phénylalanyl-L ou D-isoleucylglycyle.

A titre d'exemple, X peut être en particulier l'histamine ou un dérivé de celle-ci répondant à la formule:

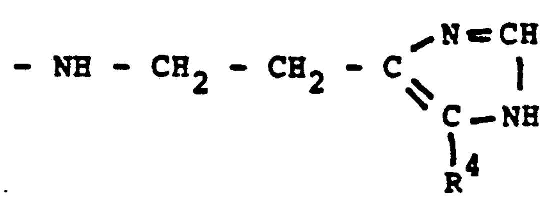

dans laquelle $R^4$ représente l'hydrogène ou $CH_3$.
X peut aussi représenter l'un des radicaux suivants:

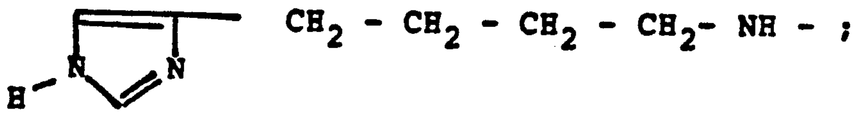

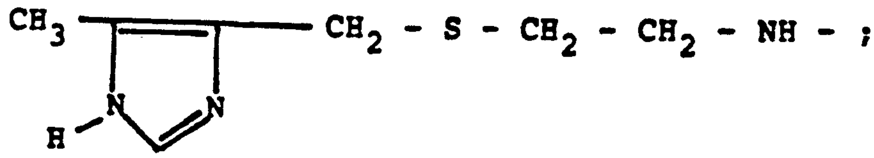

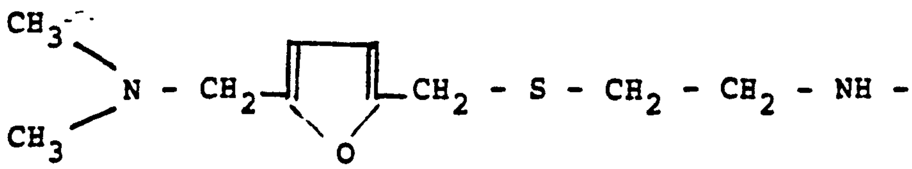

et

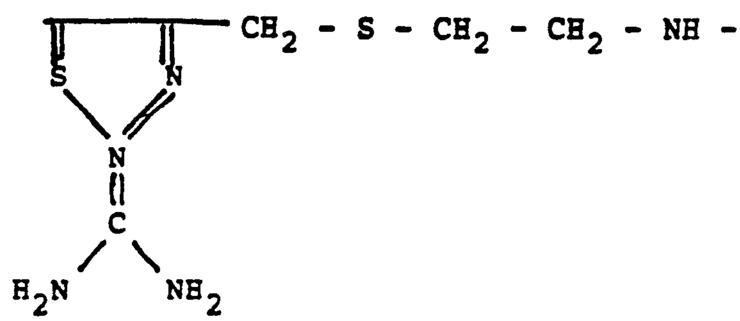

De préférence, selon l'invention, B représente le radical L-méthionyle et X représente un radical dérivé d'histamine tel que le radical de formule:

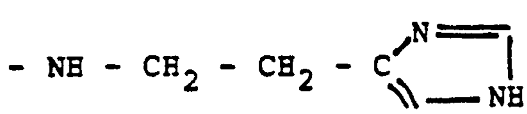

La présence d'un tel substituant permet en particulier de conférer au dérivé peptidique un effet antagoniste non seulement vis-à-vis de la gastrine et des polypeptides apparentés, mais aussi vis-à-vis de l'histamine dans ses effets au niveau du tractus digestif.

Aussi, selon un mode de réalisation préféré, le dérivé peptidique de l'invention répond à la formule suivante:

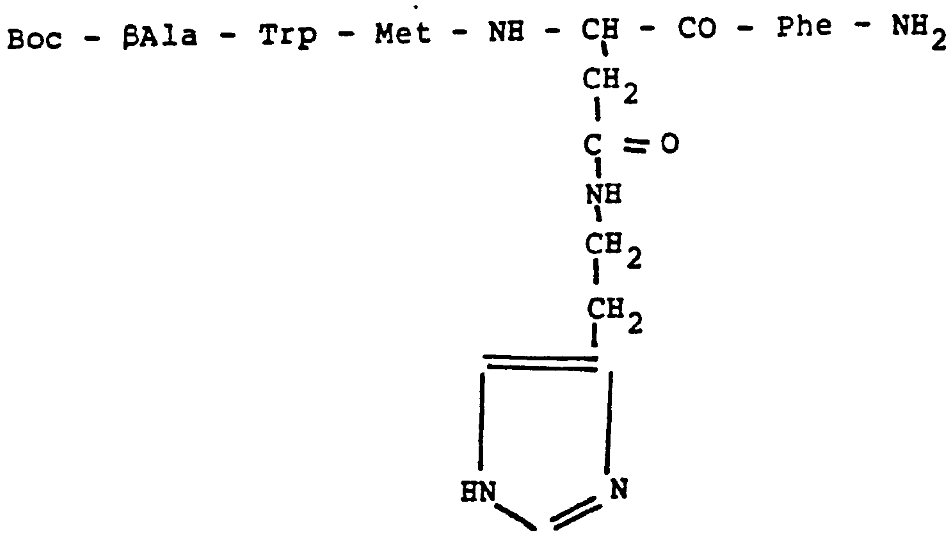

(II)

La présente invention a également pour objet un procédé de préparation du dérivé peptidique défini ci-dessus.

Ce procédé consiste à faire réagir, en présence d'un catalyseur, un composé de formule:

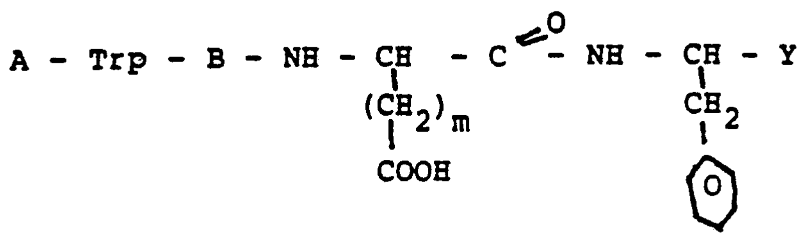

(III)

dans laquelle A, B, Y et m ont les significations données ci-dessus avec un composé de formule H-X(IV) dans laquelle X a la signification donnée ci-dessus, ou l'un de ses sels tels que le chlorhydrate, l'acétate ou le trifluoroacétate.

Les catalyseurs utilisés ont pour fonction de prévenir la cyclisation du résidu sur lequel sera accroché le groupement X. On utilise par exemple le 1-hydroxybenzotriazole ou le N-hydroxysuccinimide. Pour cette réaction, on dissout généralement le composé de formule (III) et le composé de formule (IV) dans un solvant et on ajoute également au milieu réactionnel un agent couplant pour permettre la condensation du carboxyle du composé III avec le composé IV.

Les solvants susceptibles d'être utilisés sont les solvants classiques utilisés en synthèse peptidique tels que le diméthylformamide (DMF), le diméthylsulfoxyde (DMSO), l'acétate d'éthyle, l'eau et leurs mélanges. En milieu aqueux, on s'assure que le pH reste entre 5 et 7. Si nécessaire, on ajuste le pH par addition d'une base organique telle que la triméthylamine ou la N-méthylmorpholine.

Les agents couplants susceptibles d'être utilisés sont ceux utilisés habituellement en synthèse peptidique, par exemple le dicyclohexylcarbodiimide ou le 1-cyclohexyl-3(2-morpholinoisoéthyl)carbodiimide (CMC). De préférence, pendant la réaction, on maintient le pH du milieu réactionnel à 6. Ceci peut être réalisé par addition d'une base organique telle que la triéthylamine.

Les composés de formule (III) et (IV) utilisés comme produits de depart pour préparer les peptides de l'invention peuvent être préparés par des procédés classiques. Le composé de formule (III) peut par exemple être synthétisé par la méthode de J.M. Davey, A.H. Laird et Y.S. Morley (J. Chem. Soc. C, 555, 1966).

La présente invention a également pour objet une composition pharmaceutique caractérisée en ce qu'elle comprend comme ingrédient actif un peptide répondant à la formule:

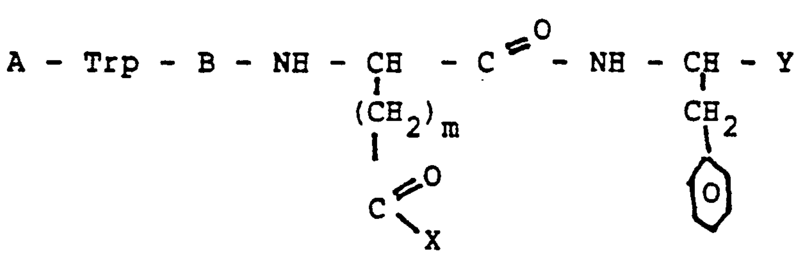

(I)

dans laquelle A, B, X, Y et m ont les significations données ci-dessus.

Les dérivés peptidiques de l'invention peuvent être utilisés comme agent de diagnostic ou comme agent thérapeutique. En particulier, ils sont actifs pour influer sur la sécrétion gastrique et pancréatique, la tonicité et la motilité gastriques et intestinales.

Les peptides de l'invention peuvent également exercer un effet:

- sur le transport ou la sécrétion d'eau et d'électrolytes sur les organes suivants: pancréas, foie, intestin grêle et glandes de Brunner;
- sur la sécrétion enzymatique (estomac, pancréas, intestin grêle)
- sur l'absorption du glucose, des électrolytes et de l'eau au niveau de l'intestin grêle;
- sur la musculature lisse, par exemple, sur les organes stimulés par la gastrine tels que le sphincter oesophagien inférieur, l'estomac, l'intestin grêle, le colon et la vésicule, ou sur les organes inhibés par la gastrine tels que le sphincter pylorique, le sphincter iléo-caecal et le sphincter d'Oddi;
- sur la circulation sanguine d'organes digestifs comme l'estomac, l'intestin grêle et le pancréas, et
- sur l'action trophique de la gastrine vis-à-vis d'un certain nombre de muqueuses ou d'organes tels que la muqueuse gastrique, la muqueuse de l'intestin grêle et le pancréas.

Les dérivés peptidiques de l'invention peuvent ainsi être utilisés comme agent thérapeutique pour le traitement d'états pathologiques tels que le syndrome de Zollinger Ellison, l'ulcère gastro-duodénal dans ses formes typiques et atypiques et l'hypersécrétion gastrique des résections intestinales.

Les compositions pharmaceutiques peuvent comprendre en plus du dérivé peptidique de l'invention un ou plusieurs diluants ou excipients acceptables du point de vue pharmaceutique et non toxiques. On peut aussi ajouter à la composition pharmaceutique d'autres principes actifs. On peut obtenir les compositions pharmaceutiques en faisant appel à des procédés classiques et à des excipients classiques. Comme composition appropriée, on peut citer par exemple les comprimés enrobés ou non enrobés, des capsules, des solutions ou suspensions aqueuses, des émulsions, des solutions ou suspensions injectables aqueuses ou non aqueuses, des poudres aptes à la dispersion et des compositions de dépôts. Les solutions aqueuses peuvent aussi contenir du diméthylsulfoxyde et/ou du chlorure de sodium.

Les compositions préférées sont des solutions ou suspensions isotoniques aqueuses injectables et stériles. Généralement, pour obtenir les effets recherchés, on administre la composition pharmaceutique de façon que la dose totale de dérivé peptidique actif, administrée en une seule fois ou en plusieurs fois soit de 1 à 200 nmol/kg/j.

D'autres caractéristiques et avantages de l'invention apparaîtront mieux à la lecture des exemples suivants donnés bien entendu à titre illustratif et non limitatif, en référence au dessin annexé, sur lequel:

- la figure 1 est un diagramme illustrant la cinétique de la réaction de formation du dérivé pentagastrine-histamine, la courbe I representant la quantité (en % molaire) de pentagastrine en fonction du temps (en h), la courbe II représentant la quantité (en % molaire) de pentagastrine-histamine formée en fonction du temps (en h) et la courbe III illustrant la quantité (en % molaire) de pentagastrine aminosuccinylée (Asc-PG) formée en fonction du temps (en h);
- la figure 2 représente le spectre d'absorption de la pentagastrine-histamine;
- la figure 3 illustre les spectres de dichroïsme circulaire de la pentagastrine-histamine, de la pentagastrine et de la pentagastrine aminosuccinylée, dans le trifluoroéthanol;
- la figure 4 illustre la réponse sécrétoire en acide chlorhydrique en fonction du temps et des produits injectés (pentagastrine et pentagastrine-histamine), lors d'essais effectués sur des rats;
- les figures 5, 6, 7 sont des courbes illustrant l'inhibition des plateaux de sécrétion stimulée par la pentagastrine par injection de pentagastrinehistamine à différentes doses, et
- la figure 8 est une courbe d'inhibition du plateau de sécrétion stimulée par l'histamine au moyen de pentagastrine-histamine ou de cimétidine.

**EXEMPLE 1**: Préparation de pentagastrine-histamine (PG-Hist)

a) - Préparation de la pentagastrine: Boc - βAla-Trp - Met - Asp - Phe - $NH_2$

On synthétise tout d'abord le tripeptide hydrazide (BOC-βAla-Trp-Met-$N_2H_2$), puis on dissout 780 mg (1,5 mmol) de ce tripeptide hydrazide dans 7 ml de diméthyl formamide (DMF) et on ajoute à la solution 1,5 mmol de $NaNO_2$ et 3 mmol de HCl à -25° C pour transformer le tripeptide hydrazide en azide. Après 15 min. d'agitation à -25° C, on neutralise la solution en ajoutant 6 mmol de N-méthylmorpholine, puis on ajoute au milieu réactionnel 1,5 mmol de dipeptide amide H-Asp-Phe-$NH_2$ dissous dans 6 ml de DMF et 1 ml d'eau. Après 16 h d'agitation à +4° C, on précipite la pentagastrine formée par addition de 200 ml d'une solution HCl 0,01 N à 0° C. Après filtration et séchage, on purifie le produit par triturations dans l'acétate d'éthyle, puis on recristallise le produit insoluble dans un mélange de 2-éthoxy-éthanol-eau (1/10 en volume). On obtient ainsi 770 mg (1 mmol) de pentagastrine (PG) dont les caractéristiques analytiques sont identiques à celles de la pentagastrine commercialisée par Imperial Chemical Industry.

b) - Préparation du dérivé pentagastrine-histamine (PG-Hist) de formule (II)

On dissout 50 mg (65,1 μmol) de la pentagastrine obtenue précédemment dans 0,3 ml de diméthylformamide (DMF) et on y ajoute 11,8 mg (64,1 μmol 1 eq.PG) d'histamine commercialisée par la Société Sigma et 10 μl d'une solution aqueuse contenant 18 μCi d'histamine $^{14}C(U)$ obtenue par décarboxylation de $^{14}C(U)$-histidine, fraîchement purifiée (RAS = 260 mCi/mmol) en tant que traceur. On ajoute ensuite au mélange 12,7 mg (94 μmol, 1,45 équivalent de PG) de 1-hydroxybenzotriazole dans 100 μl DMF en tant que catalyseur de la réaction,

et 23,5 mg (114 µmol; 1,75 équivalent PG) de dicyclohexylcarbodiimide comme agent couplant. On refroidit la solution à 0°C sous agitation magnétique. On ajuste ensuite le pH du mélange réactionnel à 6 par addition de triéthylamine. On laisse la réaction se poursuivre pendant une heure à 0°C, puis à la température ambiante. On suit le degré d'avancement de la réaction analytiquement à 280 nm par chromatographie sur couche mince (CCC) et par chromatographie liquide à haute performance (HPLC). La cinétique de formation de PG-Hist et de Asc-PG est donnée sur la figure 1 dont les courbes I, II et III illustrent les variations de la concentration (en % molaire) de chaque composé: pentagastrine (PG-courbe I), pentagastrine-histamine (PG-Hist/courbe II) et pentagastrine aminosuccinylée (Asc-PG/courbe III) en fonction du temps.

La réaction est terminée après 40 h environ. On filtre alors la dicyclohexylurée formée au cours de la réaction (solide blanc) sur un filtre Millipore LS (5 µm) et on purifie la pentagastrine-histamine sur une colonne de silice 60 d'une longueur de 28 cm et d'un diamètre de 2,3 cm en réalisant l'élution par un gradient de solvant constitué par un mélange d'acétate d'éthyle (EtoAC) et de BAW (n-butanol/acide acétique/eau avec un rapport en volume 75/10/22) dont la concentration varie de 100 EtoAC /0 BAW à 0 EtoAC/100 BAW à un débit de 4 ml/min, ce qui permet d'éluer successivement le 1-hydroxybenzotriazole en excès, la pentagastrine aminosuccinylée (Asc-PG) formée, la pentagastrine (PG) qui n'a pas réagi, puis le dérivé PG-Hist $^{14}C$ (à environ 60% de BAW). On analyse chaque pic d'élution par HPLC, comptage de la radioactivité $^{14}C$ et spectrométrie d'absorption.

La pentagastrine-histamine (PG-Hist) ainsi obtenue n'étant pas parfaitement pure, on réalise une deuxième purification sur une colonne contenant du gel LH20 (Séphadex) et ayant une longueur de 40 cm et un diamètre de 2,5 cm en utilisant comme éluant le mélange acide acétique/méthanol/eau (1/1/1 en volume) à un débit de 0,5 ml/min. La fraction correspondant à la pentagastrine-histamine (PG-Hist) est éluée avec 100 ml de solvant et liophilisée. On obtient ainsi 35,6 mg (41,4 µmol) de pentagastrine-histamine, soit un rendement global après purification de 63% de la pentagastrine de départ.

On vérifié la pureté du produit par HPLC, chromatographie sur couche mince, radioenregistrement et spectrographie. Pour la chromatographie analytique HPLC, on utilise du Partisil 10-ODS-2 avec différents solvants et différents programmes d'élution.

Le tableau 1 ci-dessous, illustre les résultats obtenus par chromatographie liquide HPLC en utilisant un débit de 1,2 ml/min et les programmes d'élution P.14, P.15 et P.16 du tableau 2 ci-dessous. Les produits sont détectés par absorption optique à 280 nm sauf pour l'histamine où la détection s'effectue à 215 nm.

TABLEAU 1

| Produit | $T_R$ Temps de rétention | | |
|---|---|---|---|
| | P.14 | P.15 | P.16 |
| PG | 17,03 | 15,70 | 15,85 |
| Histamine | | 2,86 | |
| 1-hydroxybenzotrizole | 6,76 | 5,40 | 2,90 |
| PG-histamine | 14,70 | 14,01 | 20,78 |
| BOC-Trp-Leu-Asp (Hist)Phe-$NH_2$ | | 14,50 | |

TABLEAU II

| Programme P.14 d'élution | Programme P.15 d'élution | Programme P.16 d'élution |
|---|---|---|
| 0 min 100% A 0% B | 0 min 100% A 0% B | 0 min 100% C 0% D |
| 3 min " " | 3 min " " | 3 min " " |
| 23 min 0% A 100% B | 19 min 0% 100% B | 23 min 0% C 100% D |

Dans le tableau 2, les références A, B, C et D désignent les solvants suivants:
A - Acétonitrile/tampon phosphate-triéthylamine 0,1M, pH 3,5, 20/80 (v/v),
B - 80/20 (v/v),
C - Acétonitrile/acétate d'ammonium 0,01N, pH 4,7, 20/80 (v/v),
D - 80/20 (v/v)

Sur le tableau 3 ci-dessous, on a reporté les résultats obtenus par chromatographie sur couche mince, réalisée sur plaques Si60-F254 pour les produits pentagastrine (PG) histamine (Hist), pentagastrine-histamine (PG-Hist) et le dérivé BOC-Trp-Leu-Asp(Hist)-Phe-$NH_2$.

TABLEAU III

| Produits | Rf des produits | | | Révélation |
|---|---|---|---|---|
| | E | F | G | |
| Pentagastrine | 0,83 | 0,84 | 0,31 | UV,E,TMD |
| Histamine | 0 | 0 | 0 | N,TMD,R |
| PG-Histamine | 0,30 | 0,41 | 0,42 | UV,E,TMD R |
| BOC-Trp-Leu-Asp (Hist)-Phe-$NH_2$ | | 0,45 | | UV,E,TMD R |

Sur ce tableau 3 les références E, F et G désignent les solvants suivants:
E = mélange de 20% d'acétate d'éthyle (EtOAC) et 80% du mélange (n-butanol/acide acétique/eau) ou BAW (75/10/22) (v/v/v);
F = mélange BAW 100%;
G = mélange de 60% EtOAC et 37% (v/v) de (pyridine/acide acétique/eau) ou PAW

Pour cette chromatographie, la révélation des plaques a été effectuée selon des méthodes classiques:
- par ninhydrine (N),
- par le réactif d'Ehrlich (E) (4-diméthylaminobenzaldéhyde),
- par le réactif TMD (4-4'-tétraméthyldiaminodiphénylméthane) et/ou
- par la radioactivité: R.

Sur la figure 2, on a représenté le spectre d'absorption de la pentagastrine-histamine.

Sur la figure 3, on a représenté les spectres de dichroïsme circulaire de la pentagastrine, de la pentagastrine-histamine et de la pentagastrine aminosuccinylée, en solution dans le trifluoroéthanol.

Dans le cas de la pentagastrine-histamine, l'intensité supérieure des signaux dichroïques dans la région aromatique suggère une rigidité plus importante du tryptophane. Dans l'ultraviolet lointain, l'amplitude de la bande positive centrée vers 193 nm (contribution des transitions $\pi$-$\pi^*$ des liaisons peptidiques et de la phénylalanine) est légèrement supérieure dans le cas de la pentagastrine-histamine, alors que vers 200-220 nm l'ellipticité de ce composé est plus faible.

Toutefois, l'allure générale des deux spectres (PG, PG-Hist) est peu différente, ce qui montre une similitude de conformation globale des deux peptides.

**EXEMPLE 2**

Dans cet exemple, on vérifie l'activité biologique du dérivé pentagastrine-histamine obtenu dans l'exemple 1 sur la sécrétion d'acide chlorhydrique, en utilisant le modèle du rat anesthésié à estomac perfusé selon la technique de Ghosh et Schild modifiée par Lai, Bri. J. Pharmac. 13, 54-61 (1958) et Gut. 5, 327-333 (1964).

Dans ces essais, on utilise des rats mâles de type Wistar pesant 300 ± 25 g. On prive les rats de nourriture solide pendant 18 h mais on leur laisse la possibilité de boire de l'eau à volonté, puis on les anesthésie à l'uréthane (1,25 g/kg) par voie intramusculaire. On maintient la température des rats à 34°C au moyen de lampes disposées à 60 cm environ. On introduit un cathéter de polyéthylène dans l'oesophage jusqu'au niveau du cardia et un deuxième cathéter pour recueillir la sécrétion de l'estomac au niveau du duodénum. Après lavage de l'estomac, on perfuse celui-ci en continu par une solution de chlorure de sodium à 0,9%, à un débit de 1 ml/min au moyen d'une pompe péristatique connectée au cathéter de l'oesophage.

Après une période de stabilisation du système d'environ 1 h 30 min après l'intervention, on injecte (ou on perfuse) les composés que l'on veut étudier par voie intraveineuse dans la veine du pénis et on collecte la sécrétion gastrique par fractions prélevées toutes les 20 min, l'intervalle entre ces injections étant au minimum

de 90 min. On dose l'acidité des fractions prélevées au moyen d'une solution de soude 0,01 N au virage de la phénolphtaline.

A) - Première série d'expériences: absence d'effet agoniste.

On injecte aux rats différentes doses de pentagastrine-histamine et on compare les résultats obtenus à ceux que l'on obtient lorsqu'on injecte la même quantité de pentagastrine.

La figure 4 illustre les résultats obtenus. Sur cette figure, on a porté en abscisse les doses (en nmol/kg) de pentagastrine (PG) ou de pentagastrine-histamine (PG-Hist) injectées et on a porté en ordonnée la quantité d'acide dosée dans les fractions recueillies, exprimée en µmol $H^+$ /40 min. Sur cette figure, la courbe I se rapporte à la pentagastrine-histamine (PG-Hist) et la courbe II à la pentagastrine (PG). Comme on peut le voir sur cette figure, la pentagastrine-histamine ne stimule pas de sécrétion d'acide chlorhydrique même à la dose de 62,4 nmol/kg, alors que l'activité de la pentagastrine est maximale dès 20 nmol/kg.

Ainsi, le dérivé pentagastrine-histamine n'a aucun effet agoniste. De plus, on peut en conclure que la liaison amide entre la pentagastrine et l'histamine n'est pas coupée in vivo car on observerait alors l'activité agoniste de la pentagastrine libérée.

B) - Deuxième série d'expériences: propriété antagoniste du dérivé pentagastrine-histamine vis-àvis de la pentagastrine.

Dans ces expériences, on administre aux rats répartis par groupes, une perfusion intraveineuse d'une solution isotonique contenant de la pentagastrine pour stimuler la sécrétion d'acide chlorhydrique. Lorsque la sécrétion stimulée devient stable, c'est-à-dire après environ 90 min de perfusion, on administre une dose de pentagastrine-histamine tout en maintenant la perfusion de pentagastrine.

Les résultats obtenus sont donnés sur les figures 5, 6 et 7. Sur ces figures, on a représenté différentes courbes d'inhibition du plateau de sécrétion stimulée par perfusion de pentagastrine à des doses de 0,16, 1,3 et 5,2 nmol/kg/h. Sur ces figures, on a porté en abscisse le temps et en ordonnée le pourcentage d'inhibition du plateau de sécrétion.

Les différentes courbes de la figure 5 (PG à 0,16 nmol/kg:h) se rapportent à l'inhibition obtenue avec une dose de 0,16 nmol/kg de pentagastrine-histamine (courbe 1), avec une dose de 0,64 nmol/kg de pentagastrine-histamine (courbe 2), avec une dose de 1,3 nmol/kg de pentagastrine-histamine (courbe 3) et avec une dose de 5,2 nmole/kg de PG-Hist (courbe 4).

Sur la figure 6, on a représenté les résultats obtenus lorsque le plateau de sécrétion est obtenu à une dose de 1,3 nmol/kg/h de pentagastrine. La courbe 1 se rapporte à l'inhibition obtenue par une dose de pentagastrine-histamine de 1,3 nmol/kg et la courbe 2 se rapporte à l'inhibition obtenue pour une dose de 5,2 nmol/kg/h de pentagastrine-histamine.

La figure 7 illustre les résultats obtenus lorsque la perfusion contient 5,2 nmol/kg de pentagastrine. Sur cette figure la courbe 1 se rapporte à l'inhibition obtenue à la dose de 5,2 nmol/kg de PG-Hist et la courbe 2 se rapporte à l'inhibition obtenue pour une dose de 20,8 nmol/kg de pentagastrine-histamine.

Dans le tableau 4 joint, on a reporté les moyennes (± ESM) des pourcentages d'inhibition maximales obtenues sur un groupe de n rats.

Le maximum d'inhibition (35 à 45%) est atteint lorsque la dose de PG-hist est quatre fois supérieure à celle de la pentagastrine. Si l'on augmente encore la dose de PG-Hist, la durée de l'inhibition est alors prolongée, et elle dépasse 3 h pour les doses de PG-Hist de 5,2 nmol/kg et de pentagastrine à 0,16 nmol/kg/h.

Lorsqu'on administre la pentagastrine-histamine et la pentagastrine dans un rapport 1/1, l'inhibition maximale du plateau de sécrétion varie de 15 à 25% et cette inhibition dure environ 1 h 20 min. Lorsque les deux composés sont dans un rapport PG-Hist/PG = 4, l'inhibition maximale du plateau de sécrétion atteint 35 à 45% et la durée de l'inhibition est alors d'environ 2 heures.

C) - Troisième série d'expériences; propriété antagoniste de la pentagastrine-histamine vis-à-vis de l'histamine.

Dans ce cas, on utilise le même mode opératoire que dans la deuxième série d'expériences en stimulant la secrétion par l'histamine. Dans ce cas, on injecte une dose d'histamine supérieure (13,5 µmol/kg/h). Après apparition du plateau de sécrétion, on injecte de la pentagastrine-histamine à raison de 150 nmol/kg ou à titre de comparaison 150 nmol/kg de cimétidine.

La cimétidine qui répond à la formule:

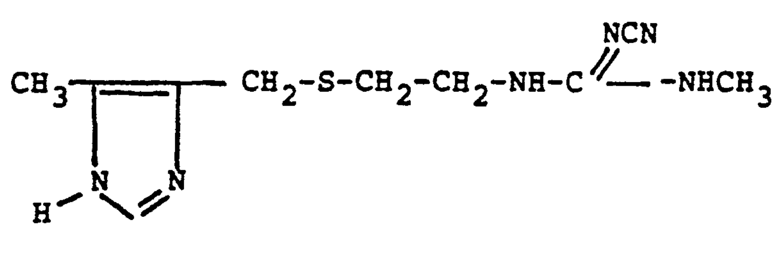

est un composé connu pour son activité antagoniste vis-à-vis de l'histamine.

La figure 8 illustre les résultats obtenus. Sur cette figure, la courbe 1 se rapporte à l'inhibition obtenue avec la cimétidine et la courbe 2 à l'inhibition obtenue avec la pentagastrine-histamine.

On observe que l'injection d'une même dose de cimétidine ou de pentagastrine-histamine entraîne une

inhibition très voisine de la réponse à l'histamine tant en intensité (environ 55% d'inhibition) qu'en durée (3 h environ).

Dans ces expériences, la moyenne (± ESM) des pourcentages d'inhibition maximale obtenue est de 56,5 ± 7,2% sur un groupe de 4 rats pour la pentagastrine-histamine (150 nmol/kg) et de 54,4 ± 2,7% sur un groupe de 4 rats pour la cimétidine à la même dose de 150 nmoles/kg.

**Tableau IV**

Moyennes (± ESM) des pourcentages d'inhibition maximale obtenue sur des groupes de n rats par injection de Pentagastrine-Histamine sur différents plateaux de secrétion de Pentagastrine.

| Doses de PG-Histamine injectées | Doses de Pentagastrine (nmoles/kg/h) en plateaux de secrétion | | |
|---|---|---|---|
| nmoles/kg | 0,16 | 1,3 | 5,2 |
| 0,16 | 25,2 ± 2,3 % n = 6 | | |
| 0,64 | 38,0 ± 1,7 % n = 6 | | |
| 1,3 | 33,6 ± 7,6 % n = 5 | 15,2 ± 6,3 % n = 7 | |
| 5,2 | 32,3 ± 7,8 % n = 11 | 46,0 ± 9,0 % n = 5 | 14,1 ± 1,2 % n = 6 |
| 20,8 | | | 35,2 ± 3,0 % n = 9 |

**Revendications**

1. Dérivé peptidique, caractérisé en ce qu'il répond à la formule suivante:

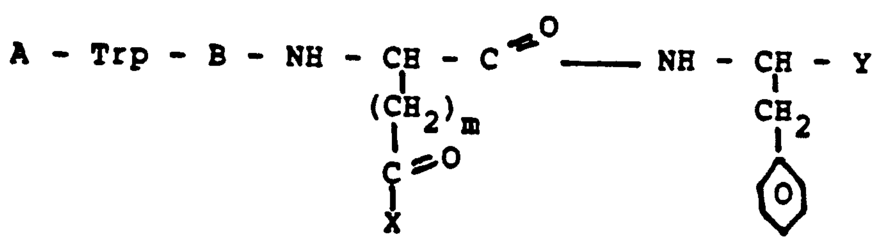

(I)

dans laquelle:

- A représente l'hydrogène; un radical dérivé d'un acide aminé choisi parmi les radicaux D ou L-pyroglutamyle, L ou D-alanyle, β-alanyle, glycyle, L ou D-prolyle, L ou D-valyle, L ou D-phénylalanyle, L ou D-homocystéyle, L ou D-aspartyle, L ou D-glutamyle, L ou D-histidyle, L ou D-méthionyle, L ou D-thréonyle, L ou D-séryle, L ou D-cystéyle, L ou D-leucyle, L ou D-isoleucyle, L ou D-arginyle, L ou D-tryptophanyle, L ou D-tyrosyle, L ou D-lysyle et L ou D-ornithyle, un groupement de formule DE où D représente le radical N-t-butyloxy-carbonyle (BOC), tert-amyloxycarbonyle (tAOC), N-benzyloxycarbonyle, N-benzoyle, N-acétyle, N-pivaloyle, N-carbamoyle ou N-succinyle et E représente une simple liaison un radical dérivé d'un acide amine choisi parmi les radicaux D ou L-pyroglutamyle, L ou D-alanyle, β-alanyle, glycyle, L ou D-prolyle, L ou D-valyle, L ou D-phénylalanyle, L ou D-homocystéyle, L ou D-aspartyle, L ou D-glutamyle, L ou D-histidyle, L ou D-méthionyle, L ou D-thréonyle, L ou D-séryle, L ou D-cystéyle, L ou D-leucyle, L ou D-isoleucyle, L ou D-arginyle, L ou D-tryptophanyle, L ou D-tyrosyle, L ou D-lysyle et L ou D-ornithyle, cet acide aminé étant non substitué ou substitué par $SO_3H$ ou un radical dérivé d'un peptide formé de 2 à 5 acides aminés choisis parmi (L ou D)-glutamyl-(L ou D)-glutamyl-(L ou D)-alanyl-(L ou D)-tyrosylglycyle, (L ou D)-alanyl-(L ou D)-phénylalanyl-(L ou D)-isoleucyl-glycyle, (L ou D)-alanyl-(L ou D)-tyrosylglycyle, (L ou D)-lysylglycyle, (L ou D)-tyrosyl-β-alanyle, (L ou D)-tyrosyl-(L ou D)-méthionylglycyle, (L ou D)-tyrosyl-(L ou D)-thréonylglycyle, (L ou D)-tyrosyl (O-sulfate)-(L ou D)-méthionylglycyle et (L ou D)-tyrosyl (O-sulfate)-(L ou D)-thréonylglycyle, ce peptide étant non substitué ou substitué par $SO_3H$;

- B représente le radical L-méthionyle, D-méthionyle, L-norleucyle, D-norleucyle, L-leucyle, D-leucyle, L-norvalyle ou D-norvalyle;

- Y représente H, $CH_2OH$, $COOR^1$ avec $R^1$ representant l'hydrogène ou un radical alkyle en $C_1$ à $C_4$ ou

$CONHR^2$ avec $R^2$ représentant l'hydrogène, un radical alkyle en $C_1$ à $C_4$ ou $NH_2$;
- m est un nombre allant de 0 à 6; et
- X représente un radical de formule:

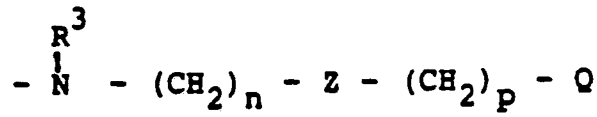

où n est un nombre allant de 1 à 8, p est un nombre allant de 0 à 3, $R^3$ représente H ou $CH_3$, Z représente une simple liaison ou S, et Q représente un radical choisi parmi les radicaux de formule:

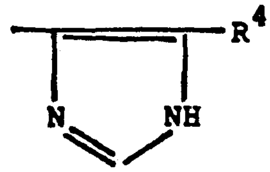

avec $R^4$ représentant H ou un radical alkyle en $C_1$ à $C_4$,

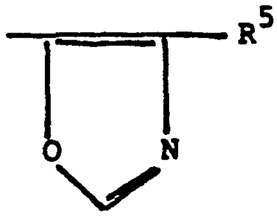 ; 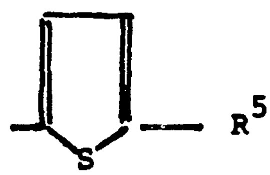 ; 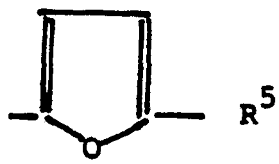

avec $R^5$ représentant H, un radical alkyle en $C_1$ à $C_4$ ou

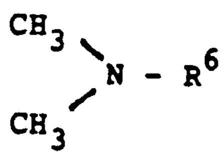

avec $R^6$ représentant un radical alkylène en $C_1$ à $C_3$; et

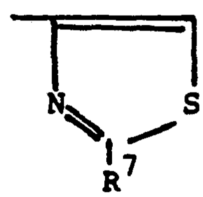

avec $R^7$ représentant H, un radical alkyle en $C_1$ à $C_4$,

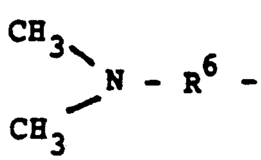

avec $R^6$ ayant la signification donnée ci-dessus, ou

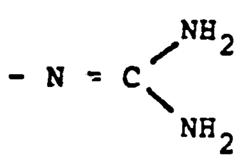

2. Dérivé peptidique selon la revendication 1, caractérisé en ce que B représente le radical L-méthionyle.

3. Dérivé peptidique selon l'une quelconque des revendications 1 et 2, caractérisé en ce que X représente le radical

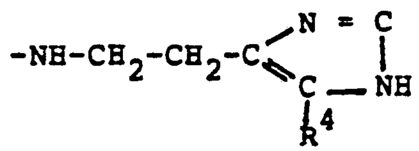

dans laquelle $R^4$ représente l'hydrogène ou $CH_3$.

4. Dérivé peptidique selon l'une quelconque des revendications 1 et 2, caractérisé en ce que X représente:

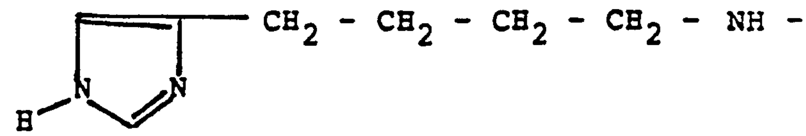

5. Dérivé peptidique selon l'une quelconque des revendications 1 et 2, caractérisé en ce que X représente:

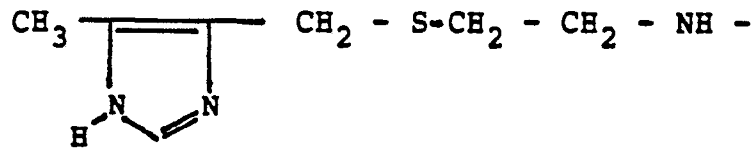

6. Dérivé peptidique selon l'une quelconque des revendications 1 et 2, caractérisé en ce que X représente:

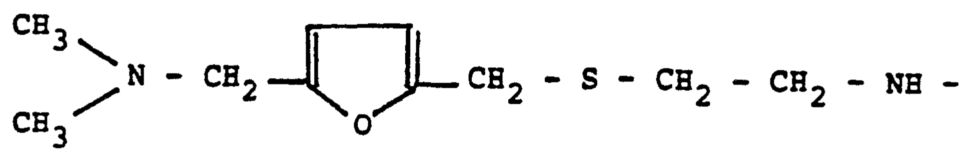

7. Dérive peptidique selon l'une quelconque des revendications 1 et 2, caractérisé en ce que X représente:

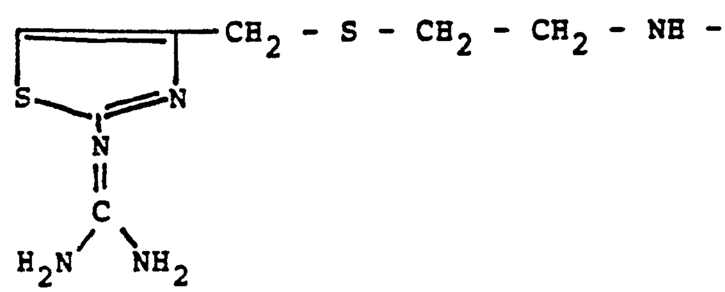

8. Dérivé peptidique de la pentagastrine selon la revendication 1, caractérisé en ce qu'il répond à la formule:

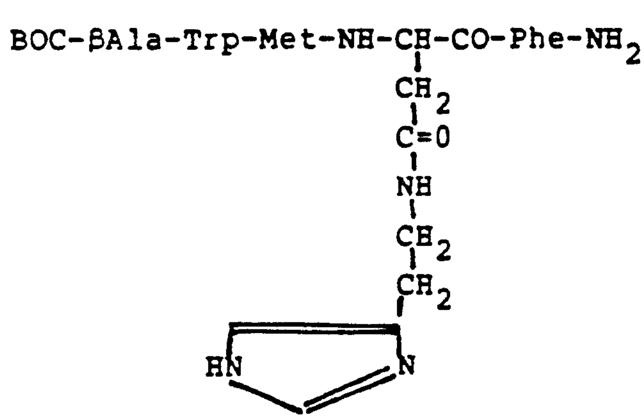

II

9. Procédé de préparation d'un dérivé peptidique selon l'une quelconque des revendications 1 à 8, caractérisé en ce que l'on fait réagir, en présence d'un catalyseur, un composé de formule:

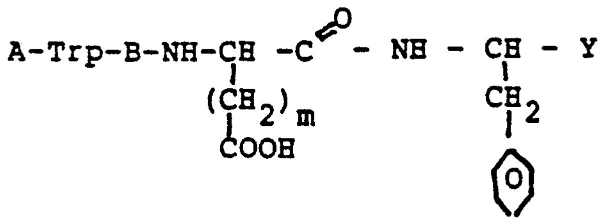

(III)

dans laquelle A, B, Y et m ont les significations données dans la revendication 1 avec un composé de formule:

(HX) IV

dans laquelle X a la même signification que dans la revendication 1, ou l'un des sels du composé HX.

10. Procédé selon la revendication 9, caractérisé en ce que le catalyseur est le 1-hydroxybenzotriazole ou le N-hydroxysuccinimide.

11. Composition pharmaceutique, caractérisée en ce qu'elle comprend comme ingrédient actif un dérivé peptidique selon l'une quelconque des revendications 1 à 8.

12. Composition selon la revenedication 11, caractérisé en ce que le dérivé peptidique répond à la formule:

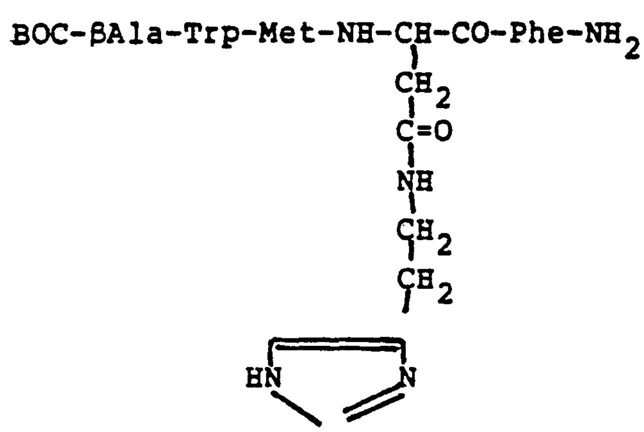

**Patentansprüche**

1. Peptidderivat, dadurch gekennzeichnet, daß es der folgenden Formel entspricht:

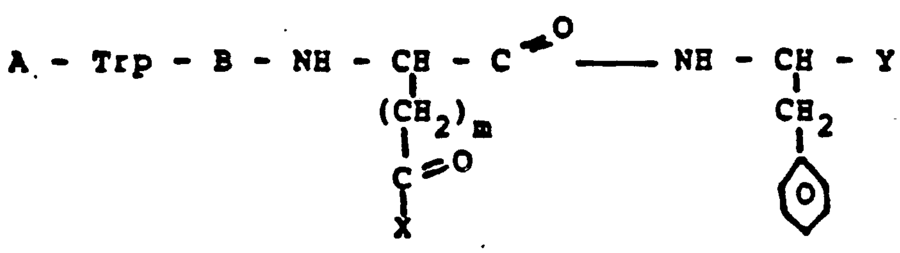

(I)

worin bedeuten:

A Wasserstoff; einen Rest, der von einer Aminosäure abgeleitet ist, ausgewählt aus der Gruppe der D- oder L-Pyroglutamyl-, L- oder D-Alanyl-, β-Alanyl-, Glycyl-, L- oder D-Prolyl-, L- oder D-Valyl-, L- oder D-Phenylalanyl-, L- oder D-Homocysteyl-, L- oder D-Aspartyl-, L- oder D-Glutamyl-, L- oder D-Histidyl-, L- oder D-Methionyl-, L- oder D-Threonyl, L- oder D-Seryl-, L- oder D-Cysteyl-, L- oder D-Leucyl-, L- oder D-Isoleucyl-, L- oder D-Arginyl-, L- oder D-Tryptophanyl-, L- oder D-Tyrosyl, L- oder D-Lysyl- und L- oder D-Ornithyl-Reste, eine Gruppe der Formel DE, worin darstellen D einen N-t-Butyloxycarbonyl (BOC)-, tert-Amyloxycarbonyl (tAOC)-, N-Benzyloxycarbonyl-, N-Benzoyl-, N-Acetyl-, N-Pivaloyl-, N-Carbamoyl- oder N-Succinyl-Rest und E

eine einfache Bindung, einen von einer Aminosäure abgeleiteten Rest, der ausgewählt wird aus der Gruppe der D- oder L-Pyroglutamyl-, L- oder D-Alanyl-, β-Alanyl-, Glycyl-, L- oder D-Prolyl-, L- oder D-Valyl-, L- oder D-Phenylalanyl, L- oder D-Homocysteyl-, L- oder D-Aspartyl-, L- oder D-Glutamyl-, L- oder D-Histidyl-, L- oder D-Methionyl-, L- oder D-Threonyl-, L- oder D-Seryl-, L- oder D-Cysteyl-, L- oder D-Leucyl-, L- oder D-Isoleucyl-, L- oder D-Arginyl-, L- oder D-Tryptophanyl-, L- oder D-Tyrosyl-, L- oder D-Lysyl- und L- oder D-Ornithyl-Reste, wobei diese Aminosäure unsubstituiert oder substituiert ist durch $SO_3H$ oder einen von einem Peptid abgeleiteten Rest, das aus 2 bis 5 Aminosäuren besteht, ausgewählt aus der Gruppe (L oder D)-Glutamyl-(L oder D)-glutamyl-(L oder D)-alanyl-(L oder D)-tyrosylglycyl, (L oder D)-Alanyl-(L oder D)-phenylalanyl-(L oder D)-isoleucyl-glycyl, (L oder D)-Alanyl-(L oder D)-tyrosylglycyl, (L oder D)-Lysylglyclyl, (L oder D)-Tyrosyl-β-alanyl, (L oder D)-Tyrosyl-(L oder D)-methionylglycyl, (L oder D)-Tyrosyl-(L oder D)-threonylglycyl, (L oder D-Tyrosyl-(O-sulfat)-(L oder D)-methionylglycyl und (L oder D)-Tyrosyl-(O-sulfat)-(L oder D)-threonylglycyl, wobei dieses Peptid unsubstituiert oder substituiert ist durch $SO_3H$;

B den L-Methionyl-, D-Methionyl-, L-Norleucyl-, D-Norleucyl-, L-Leucyl-, D-Leucyl-, L-Norvalyl oder D-Norvalyl-Rest;

Y H, $CH_2OH$, $COOR^1$, worin $R^1$ Wasserstoff oder einen $C_1$-$C_4$-Alkylrest darstellt, oder $CONHR^2$, worin $R^2$ Wasserstoff, einen $C_1$-$C_4$-Alkylrest oder $NH_2$ darstellt;

m eine Zahl von 0 bis 6; und

X einen Rest der Formel

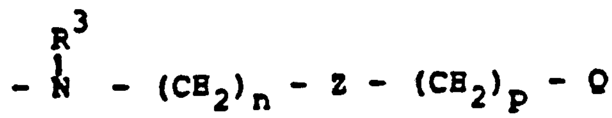

worin n eine Zahl von 1 bis 8, p eine Zahl von 0 bis 3, $R^3$ H oder $CH_3$, Z eine einfache Bindung oder S und Q einen Rest bedeuten, der ausgewählt wird aus Resten der Formel:

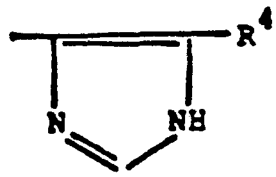

worin $R^4$ darstellt H oder einen $C_1$-$C_4$-Alkylrest,

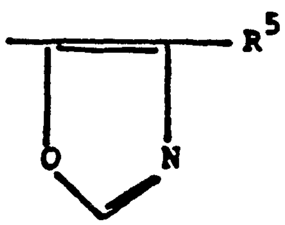 ; 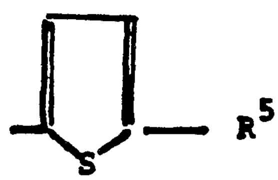 ; 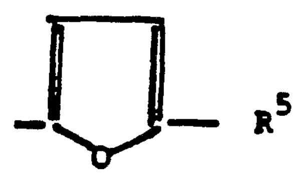

worin $R^5$ darstellt H, einen $C_1$-$C_4$-Alkylrest oder

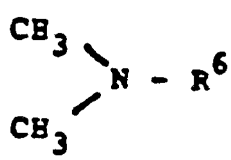

worin $R^6$ darstellt einen $C_1$-$C_3$-Alkylenrest; und

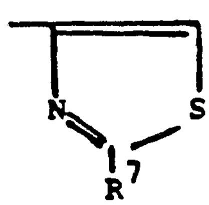

worin $R^7$ darstellt H, einen $C_1$-$C_4$-Alkylrest,

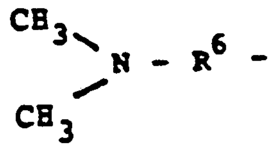

worin $R^6$ die oben angegebene Bedeutung hat, oder

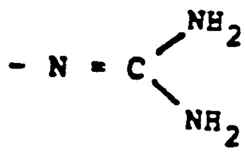

2. Peptidderivat nach Anspruch 1, dadurch gekennzeichnet, daß B den L-Methionyl-Rest darstellt.

3. Peptidderivat nach einem der Ansprüche 1 und 2, dadurch gekennzeichnet, daß X den Rest darstellt

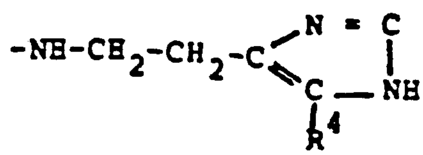

worin $R^4$ Wasserstoff oder $CH_3$ bedeutet.

4. Peptidderivat nach einem der Ansprüche 1 und 2, dadurch gekennzeichnet, daß X darstellt

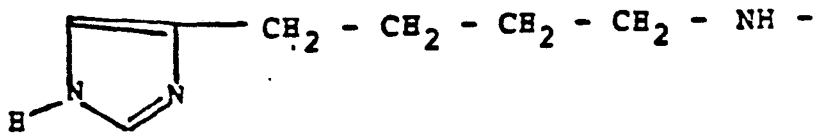

5. Peptidderivat nach einem der Ansprüche 1 und 2, dadurch gekennzeichnet, daß X darstellt

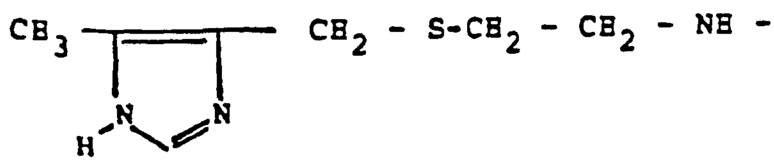

6. Peptidderivat nach einem der Ansprüche 1 und 2, dadurch gekennzeichnet, daß X darstellt:

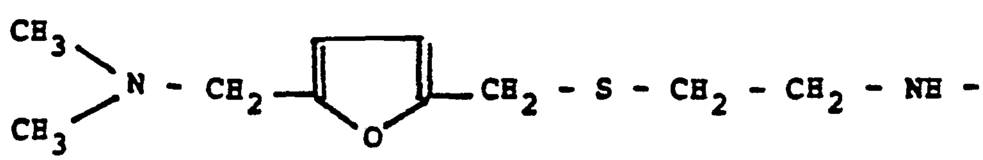

7. Peptidderivat nach einem der Ansprüche 1 und 2, dadurch gekennzeichnet, daß X darstellt

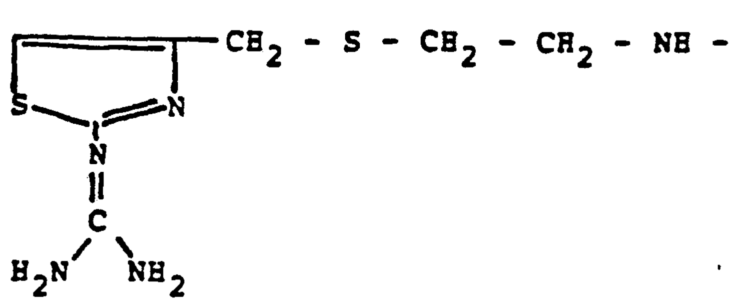

8. Peptidderivat des Pentagastrins nach Anspruch 1, dadurch gekennzeichnet, daß es der Formel entspricht:

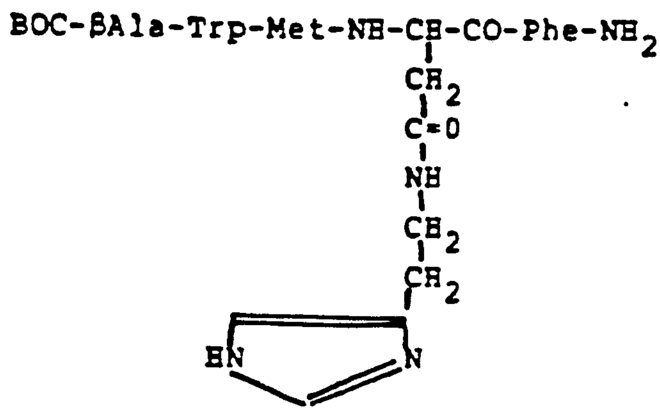

II

9. Verfahren zur Herstellung eines Peptidderivats nach einem der Ansprüche 1 bis 8, dadurch gekennzeichnet, daß man eine Verbindung der Formel

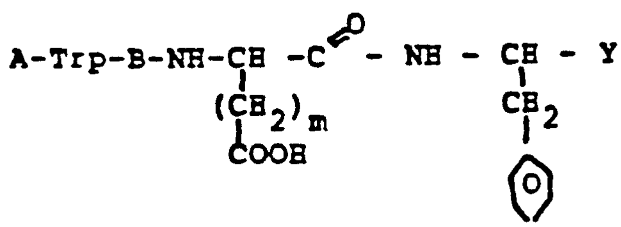

(III)

worin A, B, Y und m die im Anspruch 1 angegebenen Bedeutungen haben, in Gegenwart eines Katalysators mit einer Verbindung der Formel

(HX) IV

worin X die gleiche Bedeutung wie im Anspruch 1 hat, oder einem der Salze der Verbindung HX reagieren läßt.

10. Verfahren nach Anspruch 9, dadurch gekennzeichnet, daß es sich bei dem Katalysator um das 1-Hydroxybenzotriazol oder das N-Hydroxysuccinimid handelt.

11. Pharmazeutische Zusammensetzung, dadurch gekennzeichnet, daß sie als aktiven Bestandteil ein Peptidderivat nach einem der Ansprüche 1 bis 8 enthält.

12. Zusammensetzung nach Anspruch 11, dadurch gekennzeichnet, daß das Peptidderivat der Formel entspricht:

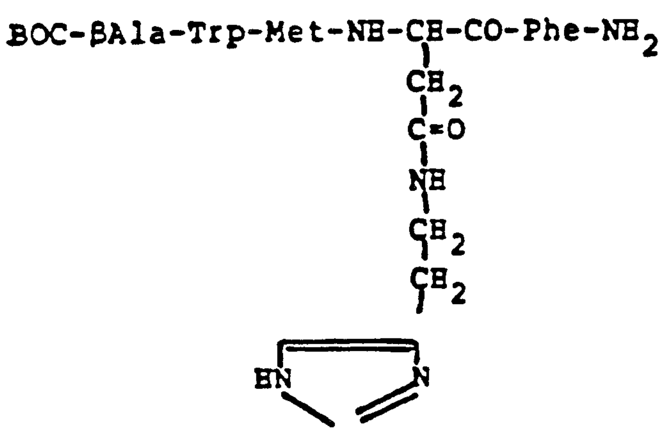

**Claims**

1. Peptide derivative, characterized in that it corresponds to the following formula:

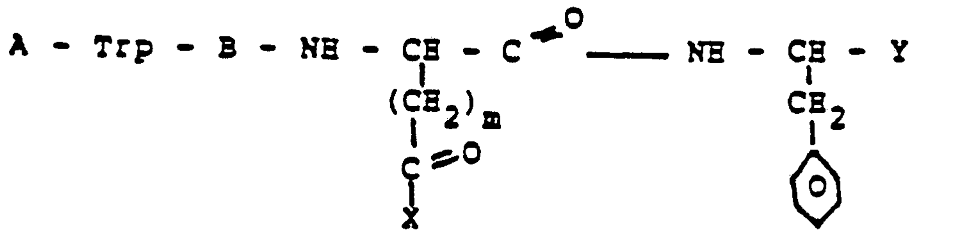

(I)

in which:

A denotes hydrogen; a radical derived from an amino acid chosen from D- or L-pyroglutamyl, L- or D-alanyl, β-alanyl, glycyl, L- or D-prolyl, L- or D-valyl, L- or D-phenylalanyl, L- or D-homocysteinyl, L- or D-aspartyl, L- or D-glutamyl, L- or D-histidyl, L-or D-methionyl, L- or D-threonyl, L- or D-seryl, L- or D-cysteinyl, L- or D-leucyl, L- or D-isoleucyl, L- or D-arginyl, L- or D-tryptophyl, L- or D-tyrosyl, L- or D-lysyl and L- or D-ornithyl radicals, a group of formula DE, where D denotes a N-t-butyloxycarbonyl (BOC), tertamyloxycarbonyl (tAOC), N-benzyloxycarbonyl, N-benzoyl, N-acetyl, N-pivaloyl, N-carbamoyl or N-succinyl radical and E denotes a single bond, a radical derived from an amino acid chosen from D- or L-pyroglutamyl, L- or D-alanyl, β-alanyl, glycyl, L- or D-prolyl, L- or D-valyl, L- or D-phenylalanyl, L- or D-homocysteinyl, Lor D-aspartyl, L- or D-glutamyl, L- or D-histidyl, L- or D-methionyl, L- or D-threonyl, L- or D-seryl, L- or D-cysteinyl, L- or D-leucyl, L- or D-isoleucyl, L- or Darginyl, L- or D-tryptophyl, L- or D-tyrosyl, L- or D-lysyl and L- or D-ornithyl radicals, this amino acid being unsubstituted or substituted with $SO_3H$, or a radical derived from a peptide consisting of 2 to 5 amino acids chosen from (L- or D-)glutamyl-(L- or D-)glutamyl-(L- or D-)alanyl-(L- or D-)tyrosylglycyl, (L- or D-)alanyl-(L- or D-)phenylalanyl-(L- or D-)isoleucylglycyl, (L- or D-)alanyl-(L- or D-)tyrosylglycyl, (L- or D-)lysylglycyl, (L- or D-)tyrosyl-β-alanyl, (L- or D-)tyrosyl-(L- or D-)methionylglycyl, (L- or D- tyrosyl-(L- or D-)threonylglycyl, ((L- or D-)tyrosyl(O-sulphate))-(L- or D-)methionylglycyl and ((L- or D-)tyrosyl-(O-sulphate))-(L- or D-)threonylglycyl, this peptide being unsubstituted or substituted with $SO_3H$;

B denotes an L-methionyl, D-methionyl, L-norleucyl, D- norleucyl, L-leucyl, D-leucyl, L-norvalyl or D-norvalyl radical;

Y denotes H, $CH_2OH$, $COOR^1$ with $R^1$ denoting hydrogen or a $C_1$ to $C_4$ alkyl radical, or $CONHR^2$ with $R^2$ denoting hydrogen, a $C_1$ to $C_4$ alkyl radical or $NH_2$;

m is a number ranging from 0 to 6; and

X denotes a radical of formula:

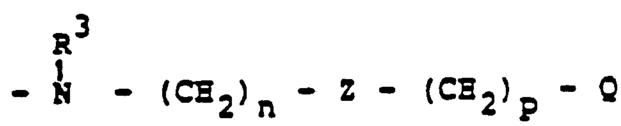

where n is a number ranging from 1 to 8, p is a number ranging from 0 to 3, $R^3$ denotes H or $CH_3$, Z denotes a single bond or S and Q denotes a radical chosen from the radicals of formula:

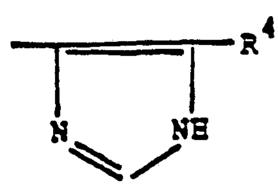

with $R^4$ denoting H or a $C_1$ to $C_4$ alkyl radical,

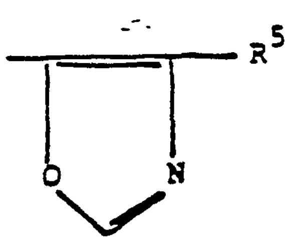 ; 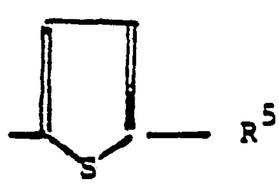 ; 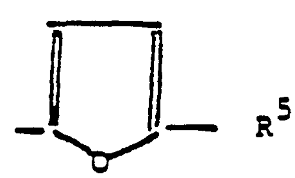

with $R^5$ denoting H, a $C_1$ to $C_4$ alkyl radical or

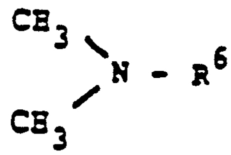

with $R^6$ denoting a $C_1$ to $C_3$ alkylene radical; and

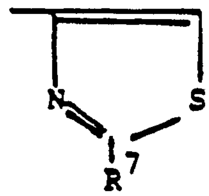

with $R^7$ denoting H, a $C_1$ to $C_4$ alkyl radical,

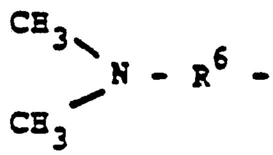

with $R^6$ having the meaning given above, or

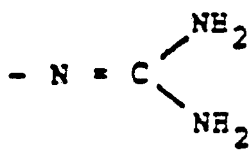

2. Peptide derivative according to Claim 1, characterized in that B denotes a L-methionyl radical.
3. Peptide derivative according to either of Claims 1 and 2, characterized in that X denotes the radical

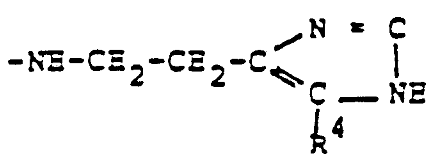

in which $R^4$ denotes hydrogen or $CH_3$.
4. Peptide derivative according to either of Claims 1 and 2, characterized in that X denotes:

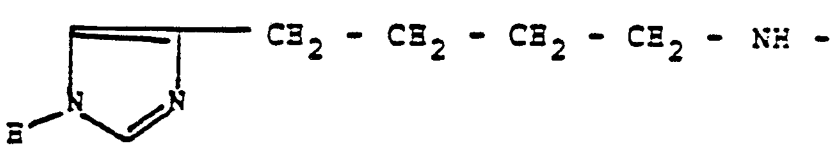

5. Peptide derivative according to either of Claims 1 and 2, characterised in that X denotes:

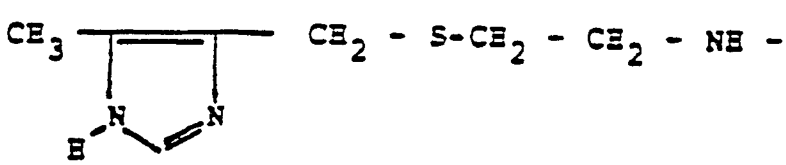

6. Peptide derivative according to either of Claims 1 and 2, characterized in that X denotes:

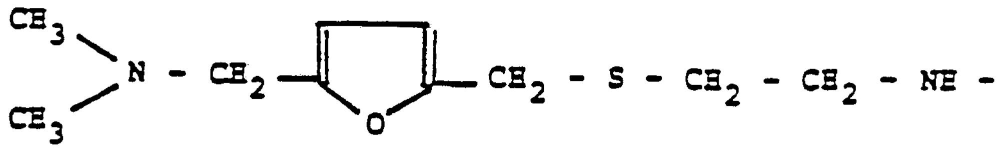

7. Peptide derivative according to either of Claims 1 and 2, characterized in that X denotes:

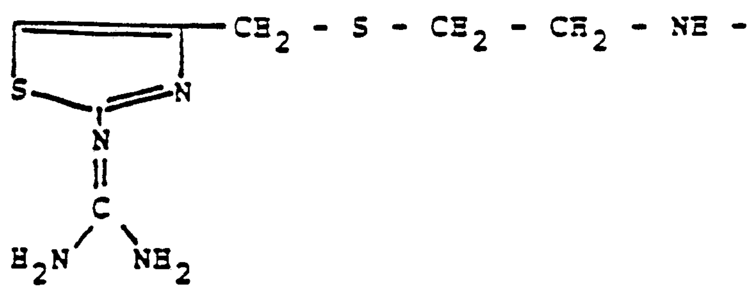

8. Peptide derivative of pentagastrin according to Claim 1, characterized in that it corresponds to the formula:

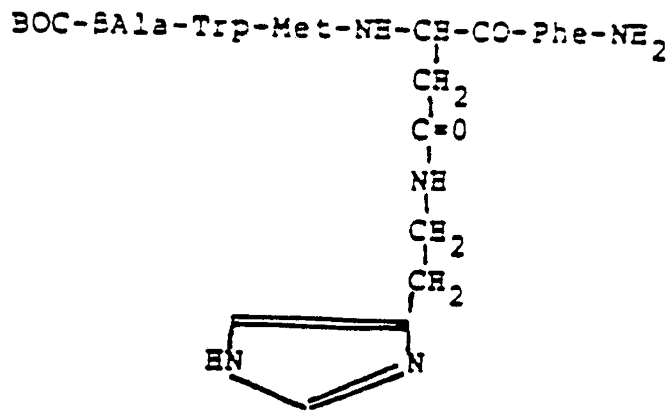

II

9. Process for preparing a peptide derivative according to any one of Claims 1 to 8, characterized in that a compound of formula:

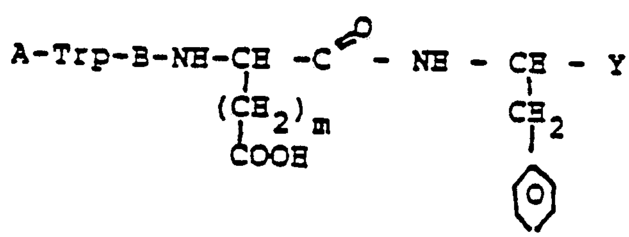

(III)

in which A, B, Y and m have the meanings given in Claim 1, is reacted, in the presence of a catalyst, with a compound of formula:

(HX) IV

in which X has the same meaning as in Claim 1, or one of the salts of the compound HX.

10. Process according to Claim 9, characterized in that the catalyst is 1-hydroxybenzotriazole or N-hydroxysuccinimide.

11. Pharmaceutical composition, characterized in that it contains as active ingredient a peptide derivative according to any one of Claims 1 to 8.

12. Composition according to Claim 11, characterized in that the peptide derivative corresponds to the formula:

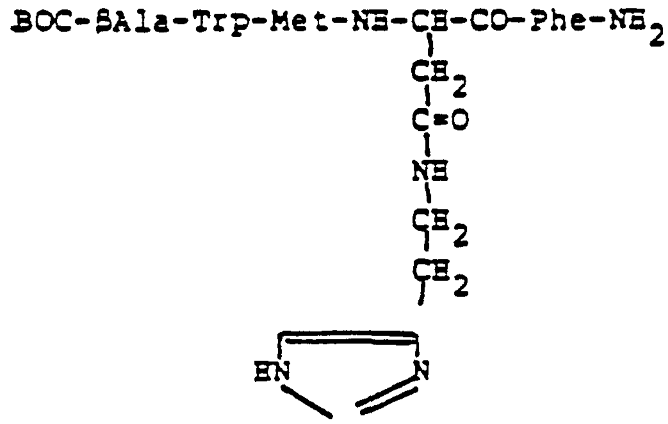

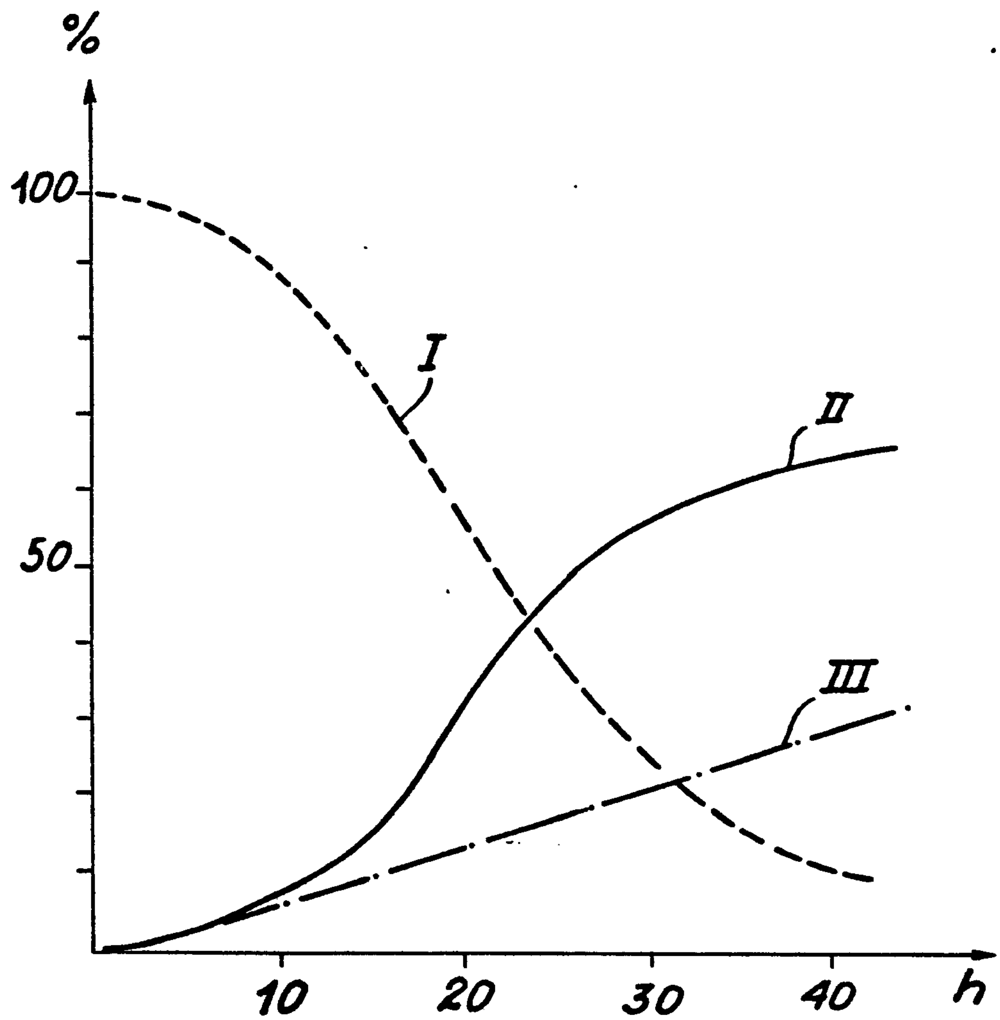


FIG.1

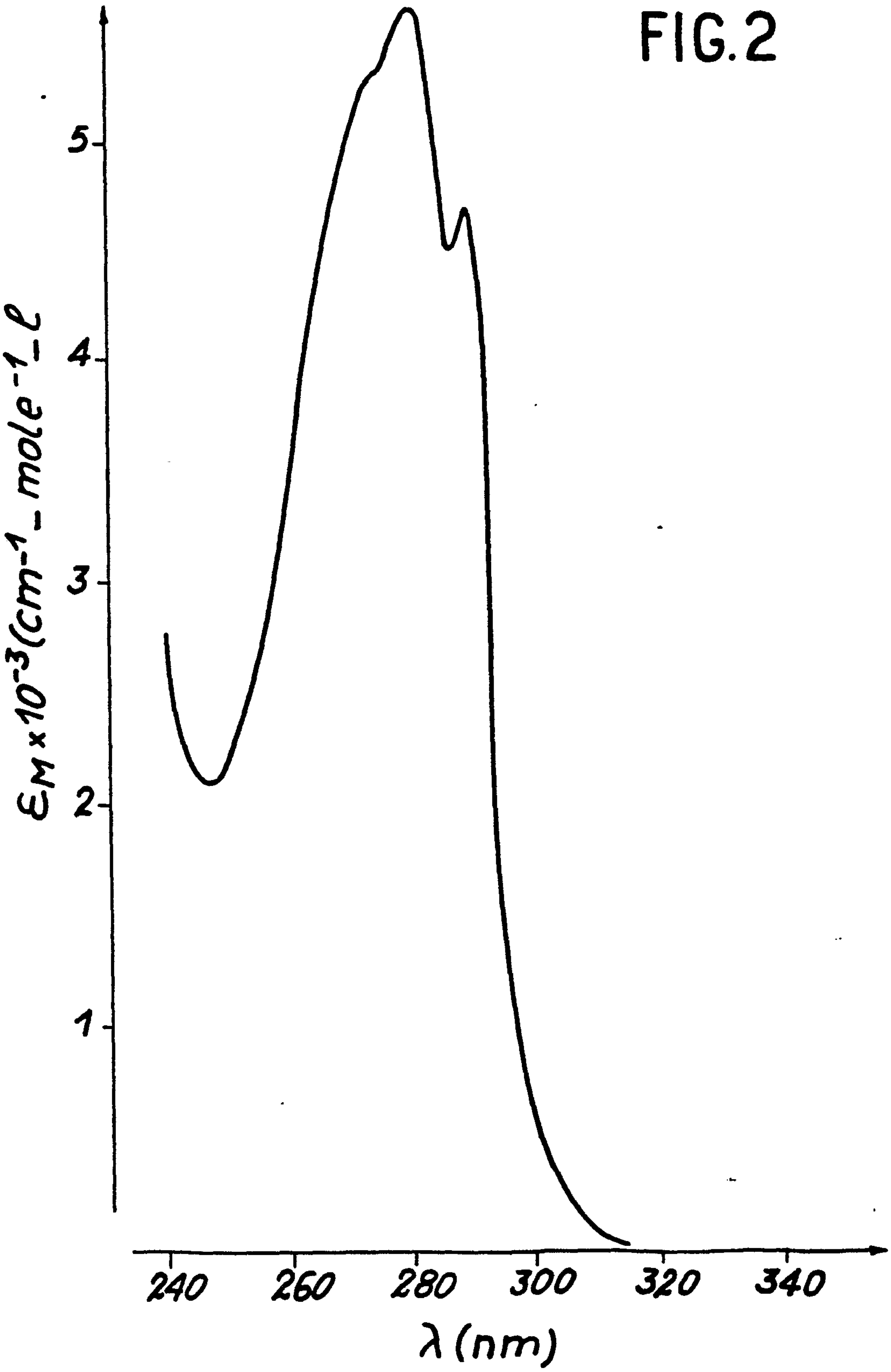


FIG.2

FIG.3

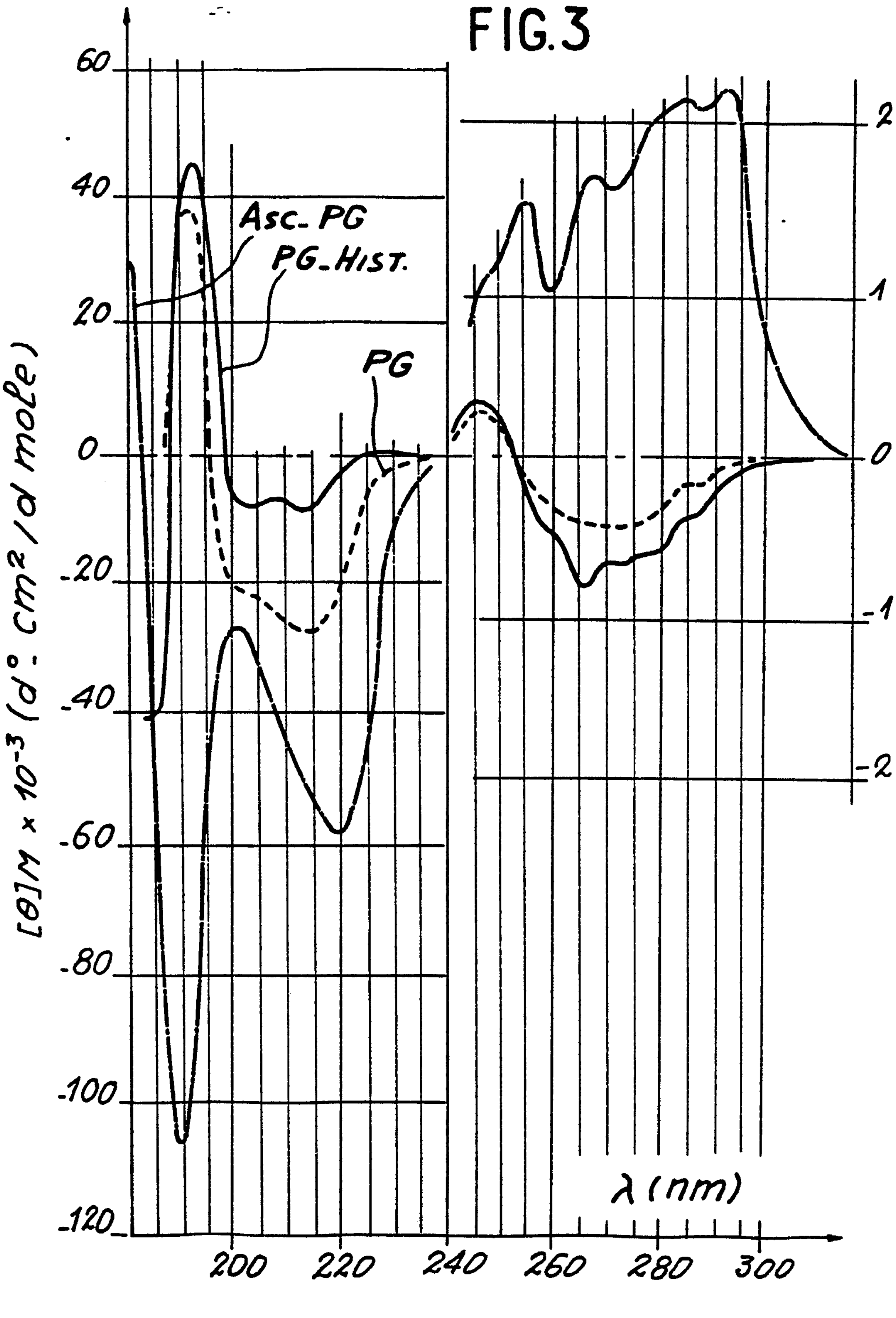
60
40
20
0
-20
-40
-60
-80
-100
-120
[θ]M × 10⁻³ (d° cm² / d mole)
Asc_PG
PG_HIST.
PG
2
1
0
-1
-2
λ (nm)
200
220
240
260
280
300

FIG.4

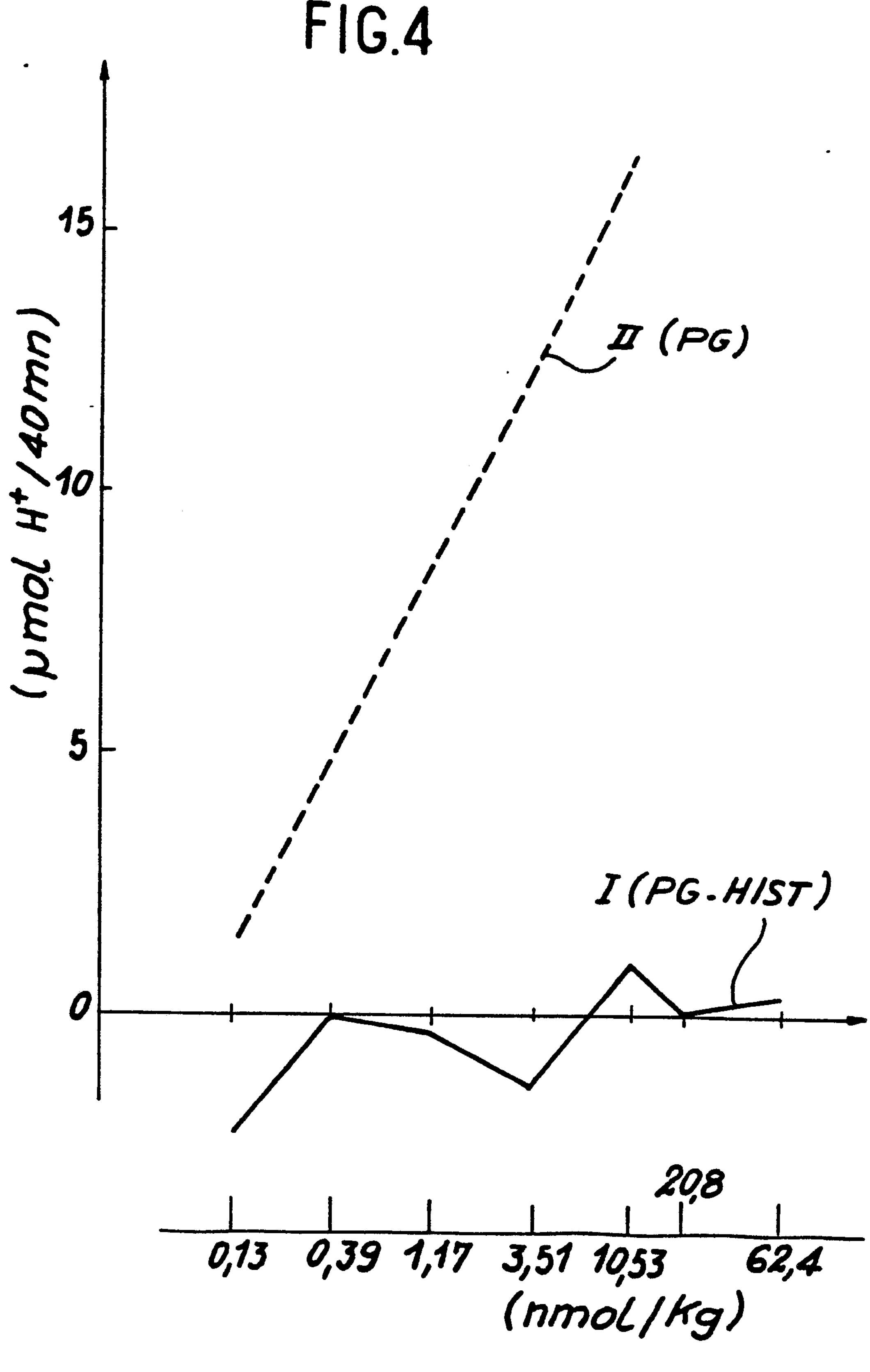
15
10
5
0
(µmol H+/40mn)
II (PG)
I (PG-HIST)
20,8
0,13
0,39
1,17
3,51
10,53
62,4
(nmol/Kg)

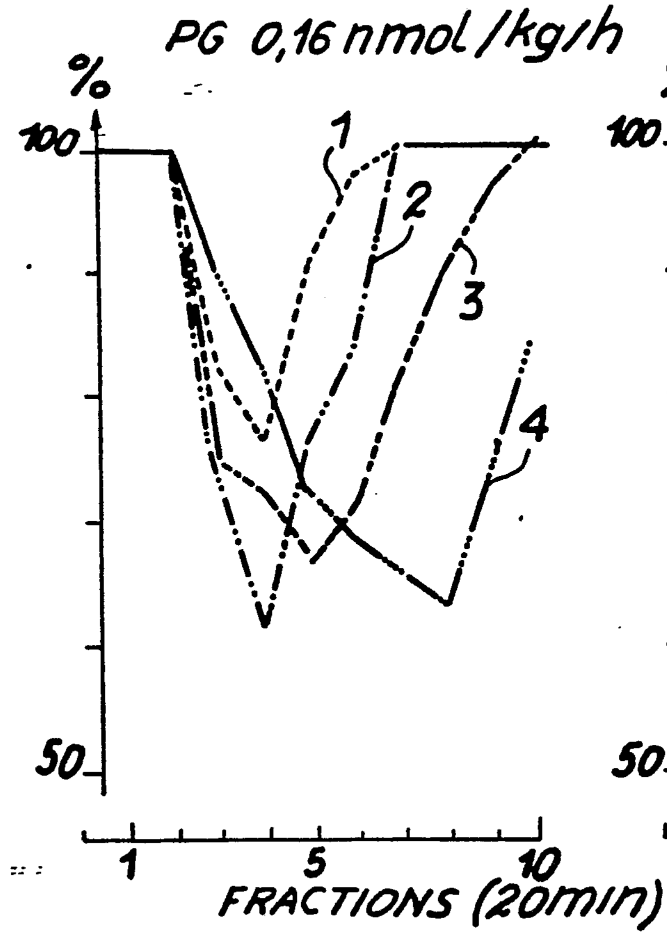


FIG.5

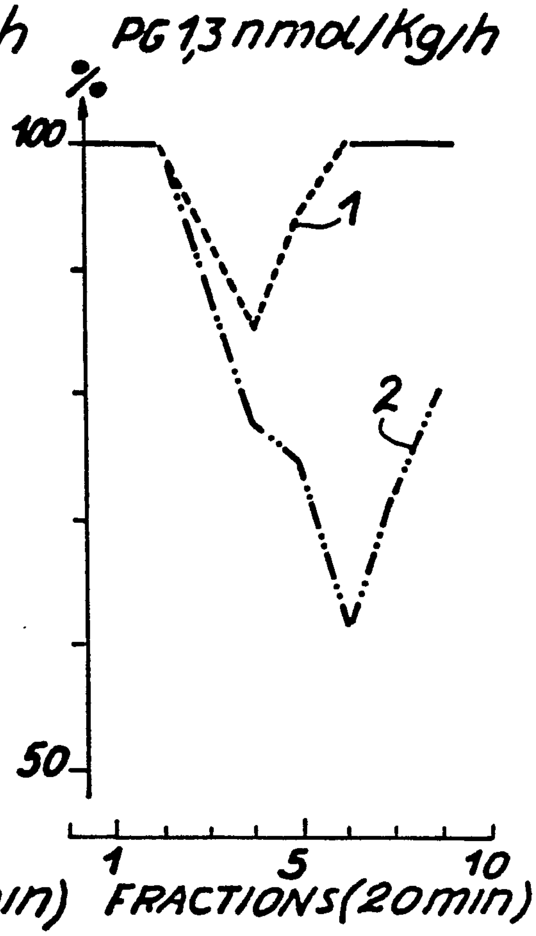


FIG.6

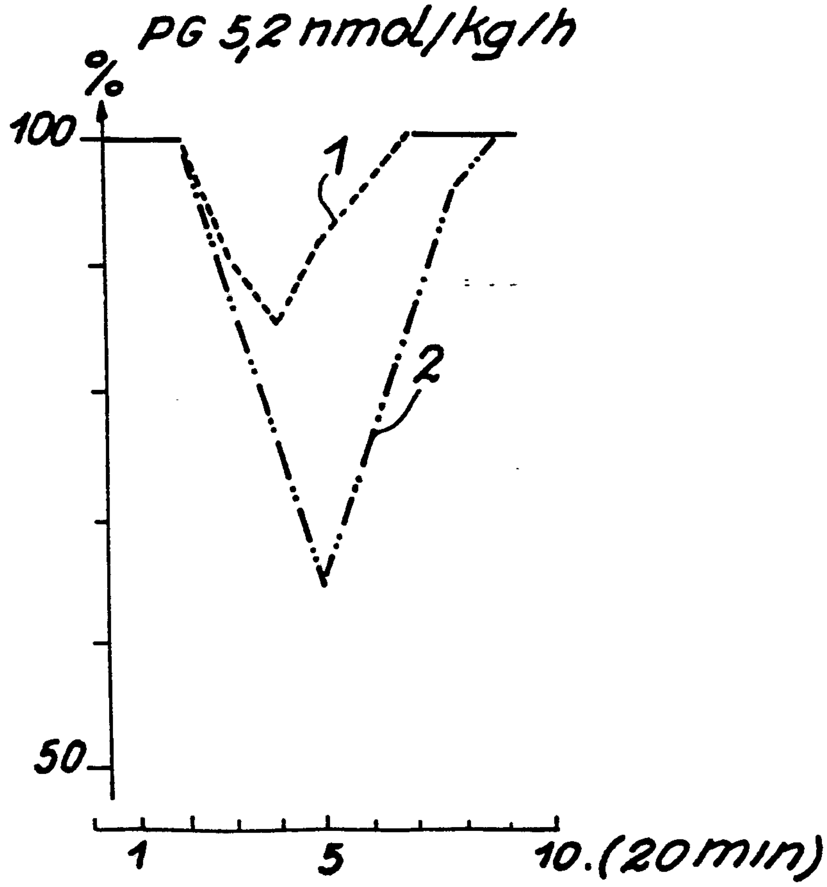


FIG.7

FIG. 8

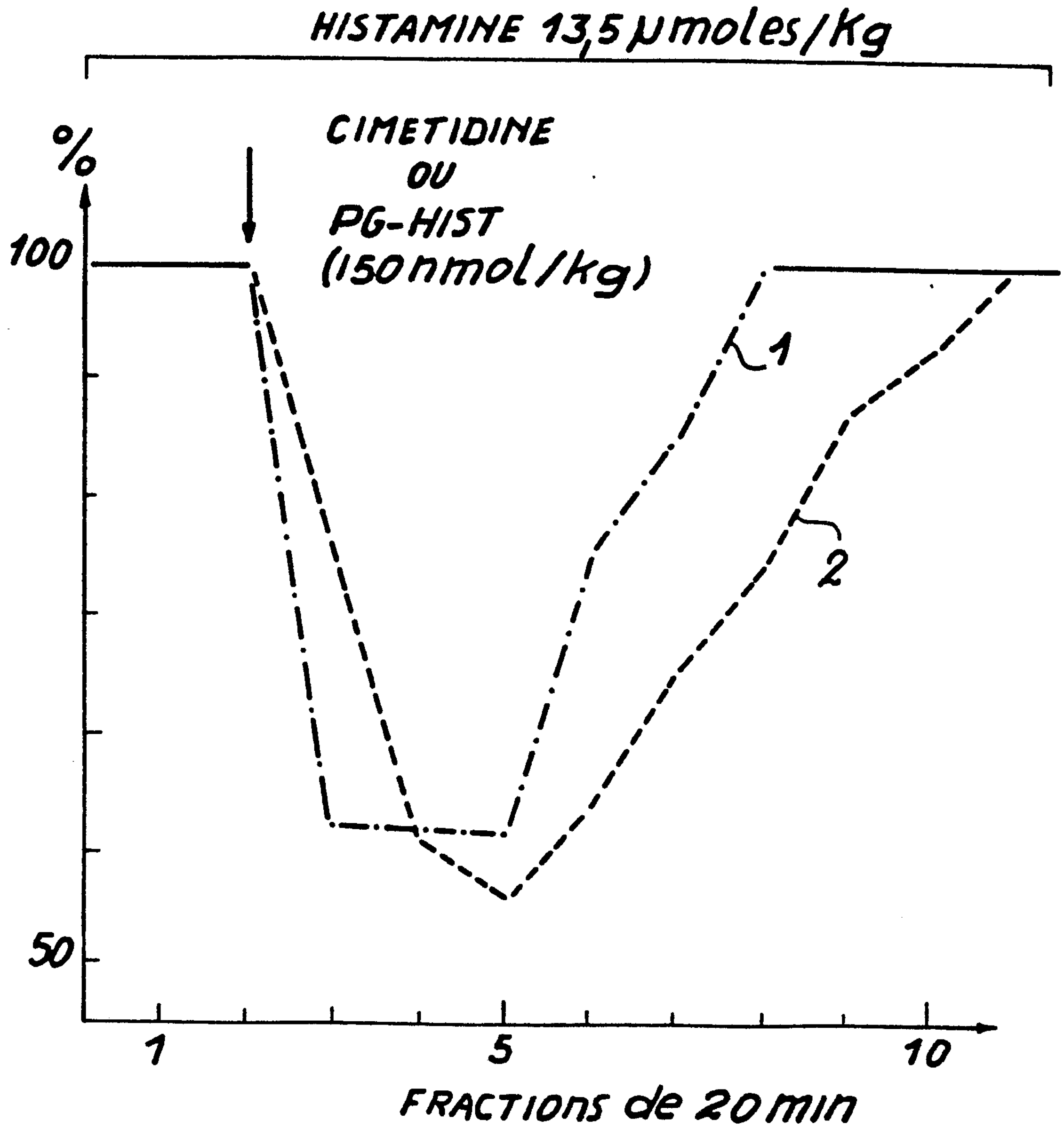

HISTAMINE 13,5 µmoles/Kg
%
CIMETIDINE
OU
PG-HIST
(150 nmol/kg)
100
50
1
2
1
5
10
FRACTIONS de 20 min